# EUROPEAN PATENT SPECIFICATION

(11) **EP 2 316 024 B1**
(45) Date of publication and mention of the grant of the patent: **29.05.2013**
(21) Application number: 09778088.6
(22) Date of filing: 25.08.2009
(51) Int. Cl.: G01N 33/487, B01L 3/00, G01N 33/543

(54) **BIOSENSOR TEST STRIP CARDS**
TESTSTREIFENKARTEN MIT BIOSENSOREN
CARTES À BANDELETTES RÉACTIVES DE BIOCAPTEUR

(30) Priority: 26.08.2008 US 198197
(43) Date of publication of application: 04.05.2011
(73) Proprietor: Roche Diagnostics GmbH, 68305 Mannheim (DE); F. Hoffmann-La Roche AG, 4070 Basel (CH)
(72) Inventor: WOOLDRIDGE, Scott, Carmel IN 46033 (US); BOLAM, Chuck, Noblesville, IN 46062 (US)
(86) International application number: PCT/EP2009/006143
(87) International publication number: WO 2010/022913

(56) References cited:
- EP-A1- 1 415 710
- WO-A1-2006/108811
- US-A- 5 053 199
- US-A- 5 366 609
- US-A1- 2005 100 880
- US-A1- 2005 247 573
- US-A1- 2007 015 983
- US-A1- 2007 134 736
- US-B2- 7 063 774

## Description

The present invention relates to biosensor test strip cards, such as are used in monitoring blood glucose.

As the number of patients suffering from diabetes and similar medical conditions increases, self-monitoring of blood glucose wherein the patient monitors his or her blood glucose levels has become a common practice. The purpose of monitoring the blood-glucose level is to determine the concentration level and then to take corrective action, based upon whether the level is too high or too low, to bring the level back within a normal range. The failure to take corrective action can have serious medical implications. Glucose monitoring is a fact of everyday life for diabetic individuals, and the accuracy of such monitoring can literally mean the difference between life and death. Failure to test blood glucose levels accurately and on a regular basis can result in serious diabetes-related complications, including cardiovascular disease, kidney disease, nerve damage and blindness.

People with diabetes who intensively manage their blood sugar experience long-lasting benefits. The Diabetes Control and Complications Trial (DCCT) was a clinical study conducted from 1983 to 1993 by the National Institute of Diabetes and Digestive and Kidney Diseases (NIDDK). The DCCT compared intensive to conventional treatments. Patients on intensive treatment kept glucose levels as close to normal as possible with at least three insulin injections a day or an insulin pump, and frequent self-monitoring of blood glucose. Intensive treatment aimed to keep hemoglobin A1c (HbA1c), which reflects average blood glucose over a 2- to 3-month period, as close to normal as possible. Conventional treatment consisted of one or two insulin injections a day with once-a-day urine or blood glucose testing. The results of the DCCT study showed that keeping blood glucose levels as close to normal as possible slows the onset and progression of eye, kidney, and nerve diseases caused by diabetes. In fact, it demonstrated that any sustained lowering of blood glucose helps, even if the person has a history of poor control.

A number of glucose meters are currently available that permit an individual to test the glucose level in a small sample of blood. Many of the meter designs currently available make use of a disposable test strip which in combination with the meter measures the amount of glucose in the blood sample electrochemically. Lot-to-lot variation during the manufacture of disposable test strips requires that the user calibrate the system for each batch of strips. Given the ramifications of accurate monitoring, improvements in the apparatus and/or procedures to meter blood glucose are desired.

US2005/0247573 discloses biosensors with reclosable cover which can be separated from an array of biosensors by perforations.

WO2006/108811 discloses a test field support structure in the form of a card with a retaining platform which when removed exposes the capillaries of the respective testing zones.

According to the present invention there is an analyte test strip card including a sheet having a plurality of biosensor test strips. Each strip is detachably connected to adjacent strips. Each strip also has at least a portion of an edge detachably connected to a retaining platform via means for separating the retaining platform extending around at least a portion of a perimeter of the sheet, wherein the retaining platform provides a surround area that defines the overall size.

In one refinement the platform is U-shaped.

In another refinement the platform includes a ROM key.

In another refinement the card is one of a plurality of cards stacked atop one another, and the stack of cards is enclosed within an air tight shrink seal.

In another refinement each of the plurality of strips has a corresponding recloseable cover overlapping a reagent portion of the strip.

In another refinement the card is substantially the size of a credit card.

In another refinement the plurality of strips are a two dimensional array of electrochemical biosensor test strips for measuring blood glucose.

In another refinement the card is in combination with a blood glucose meter having a back face, wherein the card is a blood glucose test strip card having a size permitting retention by the back face of the meter

The border zone extends around at least a portion of a perimeter of the sheet. The plurality of test strips are detachably connected to the border zone, and may include a ROM key positioned in a portion of the border zone. The ROM key includes information relating to the plurality of test strips.

Each strip in at least one row of strips may be connected to adjacent strips by a first means for separating adjacent strips and may be connected to the platform by a second means for separating strips from the platform.

The second means for separating the strips from the platform may be spaced contact points joining the strips to the platform.

The card may further include a data storage device and a BIT activated key, each key positioned in a different portion of the platform.
Figure 1 is a top view of an embodiment of a credit card sized plurality of detachably connected test strips.
Figure 2 is a top view of an embodiment of a test strip sheet having a two dimensional array of test strips and a surround area that encompasses the entire perimeter of the sheet.
Figure 3 is a top view of an embodiment of a test strip sheet having a two dimensional array of test strips and a U-shaped surround area.
Figure 4 is a top view of an embodiment of a test strip sheet having a one dimensional array of test strips and a U-shaped surround area
Figure 5 is a top view of an embodiment of a test strip sheet having a two dimensional array of test strips and a surround area with a ROM key.
Figure 6 is a top view illustrating one embodiment of a ROM key.
Figure 7 is a top view of an embodiment of a test strip sheet with a 12 count.
Figure 8 is a top view of an embodiment of a test strip sheet with a 16 count and a surround area that includes a ROM key.
Figure 9 is a top view of an embodiment of a test strip sheet with a 20 count in a vertical layout.
Figure 10 is a top view of an embodiment of a test strip sheet with a 16 count in a vertical layout and a surround area that includes a ROM key.
Figure 11 is a top view of an embodiment of a test strip sheet with a 16 count and a surround area that includes a ROM key and a BIT activated feature key.
Figure 12 is a top view of a test strip sheet with one strip detached.
Figure 13 is a side view of a combination of a test strip card retained by an analyte meter.
Figure 14 is a perspective view of a combination of a test strip card retained by the back face of an analyte meter.

For purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

Satisfying the preferences of individual diabetics with respect to management of their bG levels is important to aid compliance with treatment protocols. Discrete and easy to use products for diabetics make managing their bG levels easier and more convenient. A person with diabetes needs to have with them all items necessary to test their Blood Glucose levels. This occurs at various times while the diabetic is out and busy during the day. The diabetic wants to have bG meter test strips with them, but also wants to keep them easy to carry and discrete.

Test strips have storage requirements and come in varying storage apparatus, but most typically come in round or oval containers that are about one inch in diameter by two inches long. Other storage apparatus includes test strips in circle shaped disks (Ascensia Breeze [Bayer]) that are loaded into the meter all together. Additionally, test strips are also available individually (Medisense Pen Meter), typically individually foil wrapped test strips (e.g. Medisense Companion [Abbott]). Carrying the bG test strips individually is difficult due to strip storage requirements. At least some diabetics would prefer an easy way to carry bG meter test strips that are easy to hide, meet storage requirements and can be used individually. The existing approach has been to provide strips within the meter and/or to make use of strip packaging that is added to the meter while testing.

Various embodiments of the present invention enable packaging or cartridge footprints distinctly different than traditional oval vials, such as a substantially flat or flat storage apparatus. Refinements of such embodiments of a test strip card might further include other features as preferred. Examples of such refinements include, but are not limited to, embodiments having a data storage device (including, but not limited to, a ROM key) and/or a BIT activated feature key that provide the meter user with more functionality and information. Other refinements include using the count on the cards to drive a diabetic's testing behaviors and match the recommendations of their healthcare provider. If a patient is to test every hour, then a 12 count card might be a way of matching their day's testing needs. If a child is to test 4 times a day, then the patient or parent might use a 16 count card as a way to track the testing frequency. This might also be used as a means for doctors to track patient usage via prescriptions that identify a card count and number of cards similar to current pills. It is contemplated as within the scope of the invention that the packaging could be an individual card of strips or, alternatively, cards sold in stacks like playing cards to accommodate various strip counts. Similarly, it is further contemplated that single cards or multiple cards could be packaged in an air tight 'shrink' seal for safe shelf life.

Various embodiments of the present invention include providing multiple biosensor test strips in a sheet. The test strips are detachable from neighboring test strips. The test strips are also detachable from other fixation point(s), as when the card includes a retaining platform that borders a portion, or the entirety, of the external perimeter of the sheet. Detachability of each individual test strip from neighboring strips and/or any border area is preferably implemented via perforations or other means for separating or tearing with little to no damage to the strips. Examples of such means for separating or tearing include, but are not limited to, perforations, scoring, indentations, grooves, slots, channels, creases, and other rupturable links between the test strips. Such means for separating preferably permit a prepared separation of each strip to aid in consistent removal with little to no damage to the strips. Furthermore, such means for separating might be spaced contact points that only require a bend to 'snap' the needed strip from the rest of the strips (and/or from any surrounding perimeter of the border zone of a retaining platform if present). Thus, the perforations used might be similar to that used in store savings or coupon cards, or a credit card with smaller break-off key fob card attached.

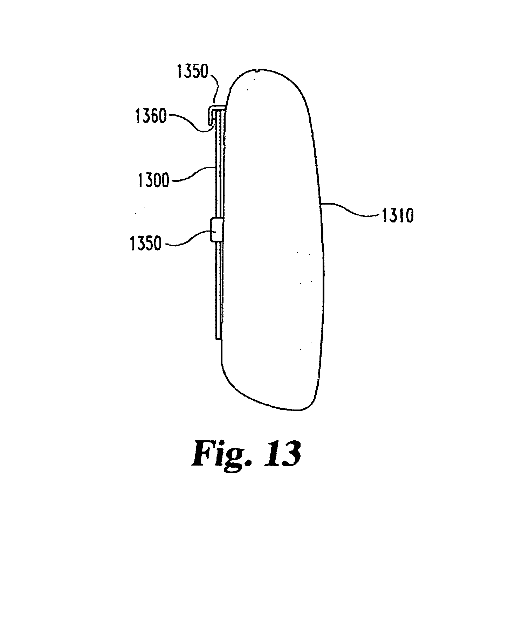

The sheet, or combination of sheet and retaining platform, is preferably, but not necessarily, approximately the size (and/or shape) of a standard credit card as, for example, defined by ISO 7810 and ISO 7813 (that define the size as 85.60 mm x 53.98 mm x .76 mm thick with 3.18 mm radius corners). Rounded corners may or may not be present as preferred. Packaging several strips within a card whose size is comparable to a credit card permits the user to store as with credit cards in personal items (wallet, purse, pockets), and to use without drawing attention to oneself because of current storage size, shape and ease of access. It will be understood that it is contemplated as within the scope of the invention that the sheet, or combination of sheet and retaining platform might be sized and shaped other than credit card sized. An example of the same is discussed below with respect to FIGs. 13-14.

The individual test strips in the sheet preferably, but not necessarily, have a recloseable protective 'film' or cover. The cover preserves the testing end of the strip prior to use, and then may also be used to cover the used test strip for disposal. Putting this protective 'film' back over the testing end after a sample has been tested aids in a more sanitary disposal of the used test strip. Furthermore, it should be understood that the protective 'film' covering does not need to be separated from the test strip when removed for using the reagent test portion, and is instead preferably recloseable. Such a protective cover is disclosed in, for example, U.S. Patent No. 7,063,774 B2 to Bhullar et al. entitled "Recloseable Biosensor" that is assigned to the assignee of the present invention and is incorporated herein by reference. The protective 'film' used to cover the end or other portion of the strip that receives blood for testing will be secured to the test strip. It will have the ability to be 'peeled' back to expose the test area and preferably also have the ability to be put back or re-applied over the tested area covering the used end with the blood sample prior to disposal. Similarly, examples of biosensors (and/or methods for manufacturing the same) for use in the present invention include, but are not limited to, U.S. Patent Nos. 5,413,690 to Kost et al. entitled "Potentiometric Biosensor And The Method Of Its Use"; 5,762,770 to Pritchard et al. entitled "Electrochemical Biosensor Test Strip"; 6,645,359 to Bhullar et al. entitled "Biosensor"; 6,662,439 to Bhullar entitled "Laser Defined Features For Patterned Laminates And Electrodes"; and 7,073,246 to Bhullar et al. entitled "Method Of Making A Biosensor"; all assigned to the assignee of the present invention, and all incorporated herein by reference.

According to the present invention the sheet of test strips includes a retaining platform. Such a platform might provide a 'surround' area or border zone that retains some or all strips until each strip is detached as, for example, being 'snapped' out. The surround area can be, for example, a "U" shape, allowing one end to be the start for removing test strips and protection for all strips not yet removed. For example, in one application the surround area might define the overall size to replicate a credit card size and/or shape (or, as previously mentioned, possess a size and shape tailored to mate with an analyte meter, such as a blood glucose meter). This allows the shape to be consistent even with the last strips (after detachment and removal of many of the strips), with some or all of the strips remaining protected and less likely to be lost. Alternatively, the 'surround' might define one or more (two being an example) polygon space(s), particularly a rectangle. Each such space receiving within it a sheet of test strips and the strips being detachably connected to the surround area as well as to neighboring strips.

It should further be understood that it is contemplated as within the scope of the invention that the border zone or 'surround' area can be used for advertisement, barcode, data storage device, BIT activated feature set key device, company logos and/or other product information. Such data storage device and/or BIT activated key might be detached via the previously described snap off mechanism, or may be connected to the card via a connection that permits reconnection to the border zone of the card.

The data storage device can be used, for example, to calibrate the meter with respect to any lot to lot variation of the test strips of the card while still attached to the card. Alternatively, the data storage device might be removed from the card for insertion and retention within the meter. In one refinement, each test strip card includes a ROM key, even when several test strip cards are bundled together. In yet another alternative, a single data storage device is included with the lead card for a 'pack' of cards. In yet another alternative, a data storage device might be separate from a single card or a 'pack' of cards. Each of these different possibilities allow for multiple count packaging, thus permitting a combination of cards 'bundled' for sale and customer needs. The data storage device might also include information relating to the test strips other than meter calibration information, such as information calibrating the number of test strips associated with a card or 'pack' of cards. The data storage device might be, for example, some type of ROM key, some other type of integrated circuit memory chip, a three dimensional or two dimensional bar code, or a swipable magnetic strip, or even an RFID chip. Examples of a ROM key data storage device and types of data stored thereon include those disclosed in U.S. Patent Nos. 5,053,199 to Keiser et al. entitled "Electronically Readable Information Carrier" and 5,366,609 to White et al. entitled "Biosensor Meter With Pluggable Memory Key", both assigned to the assignee of the present invention, and both incorporated herein by reference.

The BIT activated feature set key can provide the means for several options of information, demonstrations and/or interactive programs to be run on the meter device. As an example, the Accu-Check Advantage 3 meter from Roche Diagnostics Corporation, an affiliate of the assignee of the prevent application, had a bit activated feature set 'ROM key' type of chip that could be used with the meter. In one or more embodiments of the present invention a BIT activated feature set key could be used for tutorials, owner's and/or user's manuals for the meter, other reference information and interactive games to be displayed and run on the meter. All of these and more options might be programmed into the BIT activated feature set chip and used on the bG meter. Thus, the test strip cards could have short movies, games or instructional items or videos, and the bG meter would receive the key and display or show the same on the meter 'screen.'

Discrete carrying of test strips in, for example, a 'credit card' standardized shape provides a solution for diabetics that want a substantially flat or flat method of storage that fits compartments they already have available to them. That is to say, storage in multiple types of personal carry along items (e.g.: wallet, purse, pockets). At least some of the embodiments of the invention described herein are also easy for the diabetic consumer due to the 'break-off', or 'snap-out' method of removing individual test strips. Such connects the common consumer to familiar concepts that are already present in the consumer's world outside of medical evaluation, such as store cards and discount cards that often include a bar code, or other encoded transaction cards or tags.

It will be understood that that biosensor test strips other than electrochemical test strips are contemplated as within the scope of the invention, such as, for example, optical test strips. It will further be understood that the test strips are not limited to use in testing blood glucose, and that testing of other analytes is contemplated as within the scope of the invention.

All of the above and other aspects will now be discussed further below with respect to specific examples illustrated in the figures.

While meters have grown smaller and easier to carry, the diabetic still needs to carry a container of strips that is approximately one inch in diameter by about two inches long, or carry strips individually that may have unique storage needs. Thus, various embodiments of the present invention have the test strips in a sheet. The sheet and/or associated retaining platform are preferably, but not necessarily, about the size of a credit card for ease of carrying and storage discretely. Each test strip is removable for individual use.

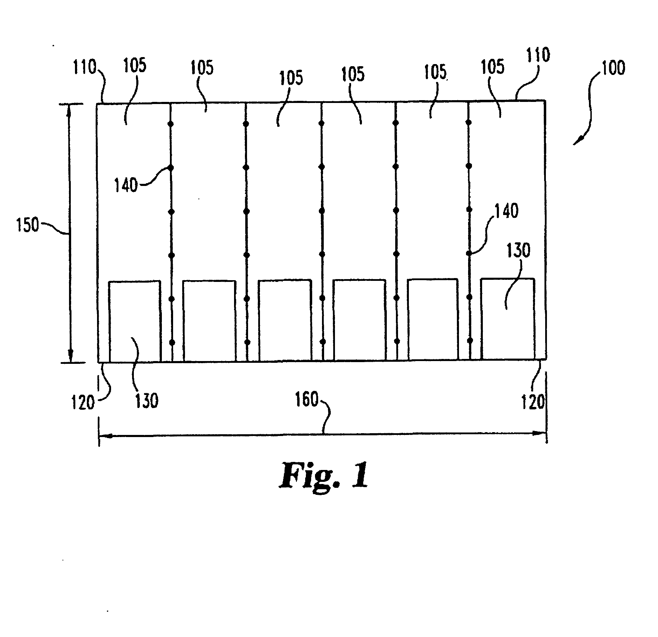

With reference to FIG. 1 there is illustrated a sheet 100 that includes a plurality of test strips 105 aligned in a one dimensional array to replicate a credit card size. The plurality of test strips extend between a first end 110 and a second end 120. A recloseable cover 130 overlaps a reagent portion (not illustrated in FIG. 1) of each test strip. The reagent portion is substantially adjacent second end 120. The test strips 105 are detachably connected to adjacent test strips. FIG. 1 illustrates with 'dots' 140 how perforations might be between adjacent test strips 105. The sheet 100 has a height 150 and a width 160. Height 150 is preferably approximately the height (53.98 mm) of a credit card. Width 160 is preferably approximately the width (85.60 mm) of a credit card.

The count of the card illustrated in FIG. 1 is six test strips. However, it should be understood that the count of this card and other cards illustrated herein may be altered by selection of the size of each test strip and how the test strips are arrayed. For example, while various embodiments are preferably credit card sized, other sizes are contemplated as within the scope of the invention unless such feature is specifically claimed. Test strips manufactured by Roche Diagnostics Operations Inc., the assignee of the present application, typically are 38 mm long and have widths of 7 mm or 9 mm. Thus, using the standard sizing of Roche Diagnostics Operations Inc. test strips having a 7 mm width, packaging test strips to replicate the size of a credit card will allow for at least ten test strips within the standard overall size. It should be understood that it is contemplated as within the scope of the invention that different lengths and/or widths of test strips might be utilized and arrayed to result in a desired count. Alternatively, the size of the test strip card might be enlarged or shrunk depending on the desired count. In yet another alternative, both the dimensions of the card and test strips might be altered to achieve the desired count.

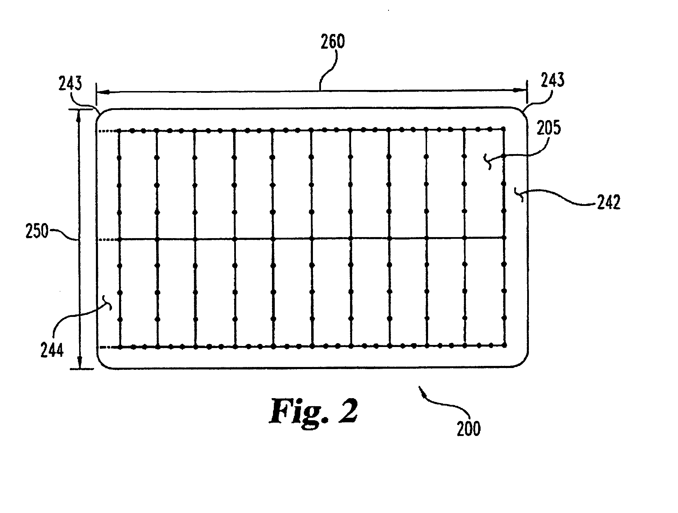

With reference to FIG. 2 there is illustrated another embodiment of a test strip card 200 having a plurality of electrochemical biosensor test strips 205 in a two dimensional array. In this embodiment the card 200 includes a surround area 242. Surround area 242 may be U-shaped, or alternatively might include (as illustrated in FIG. 2) optional area 244. Alternatively, instead of optional area 244 being present as part of surround area 242, it might instead be replaced by additional test strips. The card 200 has a height 250 and a width 260. Such dimensions preferably, but not necessarily, corresponding to those of a credit card. Surround area 242 might further include rounded corners 243. The card 200 includes first means for separating (not illustrated in FIG. 2) adjacent test strips 205 from one another as well as second means for separating (not illustrated in FIG. 2) test strips 205 from the surround area 242. The first means for separating and/or the second means for separating might each be any of the previously described mechanisms to permit singulation of an individual test strip. While not illustrated, test strips 205 have a reagent test portion and might also include a recloseable cover. The count of the card illustrated in FIG. 2 is sixteen test strips.

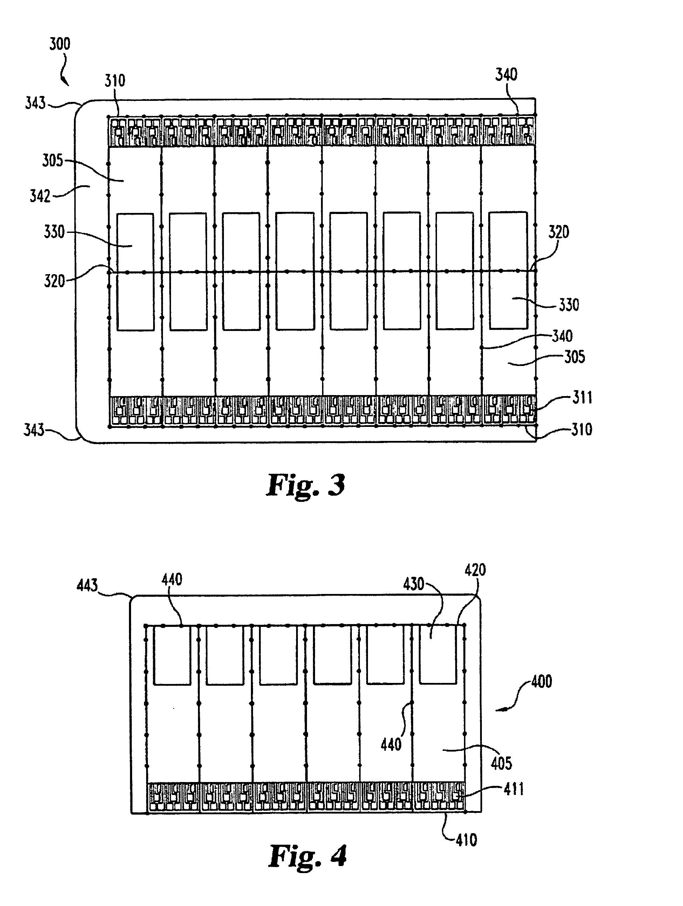

With reference to FIG. 3 there is illustrated another embodiment of a test strip card 300 having a plurality of electrochemical biosensor test strips 305 in a two dimensional array. In this embodiment the card 300 includes a U-shaped surround area 342 with rounded corners 343. The legs of the U-shaped surround area 342 extend along the width of the card 300. The card 300 preferably has a height and a width corresponding to those of a credit card. The card 300 includes first means for separating 340 adjacent test strips 305 from one another as well as second means for separating 340 test strips 305 from the surround area 342. The means for separating 340 are preferably perforations in the edges of adjacent test strips that are only joined at connecting points 340. Each test strip extends between a first end 310 and a second end 320. A recloseable cover 330 overlaps a reagent portion (not illustrated in FIG. 3) of each test strip 305. The reagent portion is illustrated as substantially adjacent or adjacent to second end 320. The first end 310 preferably includes a gripping portion 311 for the user to grasp in detaching (by, for example, bending to snap off) test strips 305 from both adjacent test strips 305 and surround area 342. The count of the card illustrated in FIG. 3 is sixteen test strips.

With reference to FIG. 4 there is illustrated another embodiment of a test strip card 400 having a plurality of electrochemical biosensor test strips 405 in a one dimensional array. In this embodiment the card 400 includes a U-shaped surround area 442 with rounded corners 443. The legs of the U-shaped surround area 442 extend along the height of the card 400. The card 400 preferably has a height and a width corresponding to those of a credit card. The card 400 includes first means for separating 440 adjacent test strips 405 from one another as well as second means for separating 440 test strips 405 from the surround area 442. The means for separating 440 are preferably perforations in the edges of adjacent test strips that are only joined at connecting points 440. Each test strip extends between a first end 410 and a second end 420. A recloseable cover 430 overlaps a reagent portion (not illustrated in FIG. 4) of each test strip 405. The reagent portion is illustrated as substantially adjacent or adjacent to second end 420. The first end 410 preferably includes a gripping portion 411 for the user to grasp in detaching (by, for example, bending to snap off) test strips 405 from both adjacent test strips 405 and surround area 442. The count of the card illustrated in FIG. 4 is six test strips.

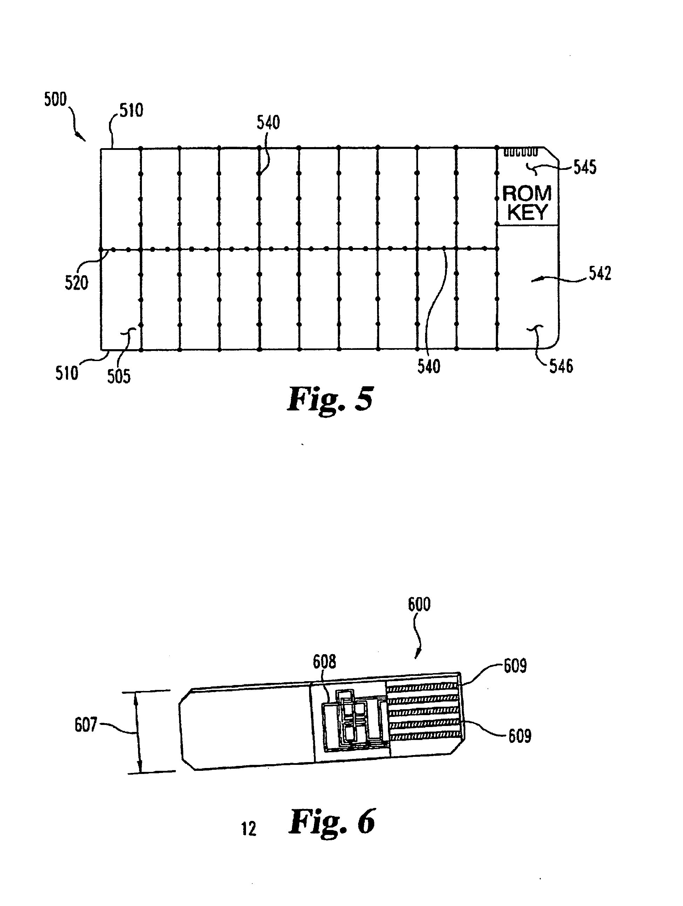

With reference to FIG. 5 there is illustrated another embodiment of a test strip card 500 having a plurality of electrochemical biosensor test strips 505 in a two dimensional array. In this embodiment the card 500 includes a surround area 542 at only one end of the card 500. The surround area 542 has a first portion 545 that includes a data storage device and a second portion 546. Second portion 546 might include manufacturer logo, product information, advertisements, and/or a BIT feature key. The card 500 preferably has a height and a width corresponding to those of a credit card. The card 500 includes means for separating 540 adjacent test strips 505 from one another as well as means for separating 540 test strips 505 from the surround area 542. The means for separating 540 are preferably perforations in the edges of adjacent test strips that are only joined at connecting points 540. Each test strip extends between a first end 510 and a second end 520. While not illustrated, the test strips 505 have a reagent test portion and might also include a recloseable cover. The count of the card illustrated in FIG. 5 is twenty test strips.

ROM keys sold with existing products are typically a 'chip' that is 'pressed' or added to a plastic holder that accommodates the mating 'slot' on the meter. In embodiments of the present invention, however, a separate plastic holder is not required. For example, in one embodiment the data storage device could be manufactured utilizing critical traces contained with laser etched configurations that burn away contacts as on a detachable strip and then 'heat stack', 'sonic weld' or 'bond' the chip circuit board to the retaining platform so that the data storage device is attached like a detachable test strip. With reference to FIG. 6 there are illustrated further aspects of one embodiment of a data storage device 600. Data storage device 600 might include a thickness 607 that is approximately the same as the typical strip thickness. Data storage device includes a section 608 that has chip functionality and a plurality of connection points 609.

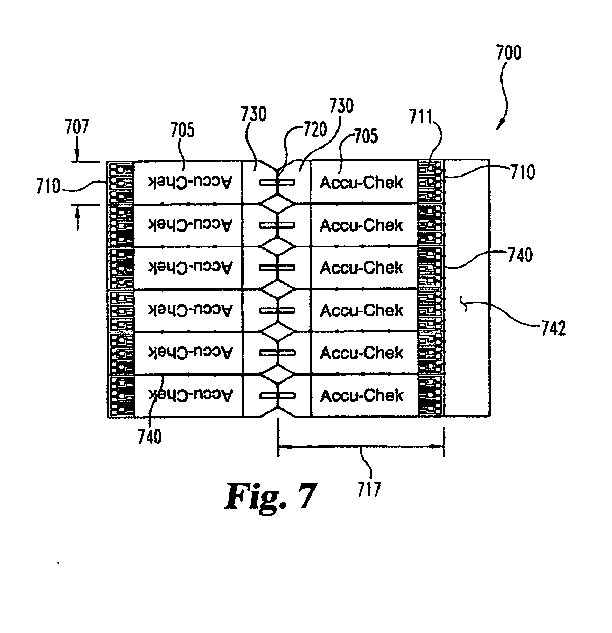

With reference to FIG. 7 there is illustrated another embodiment of a test strip card 700 having a plurality of electrochemical biosensor test strips 705 in a two dimensional array. In this embodiment the card 700 includes a surround area 742 at only one end of the card 700. The card 700 includes means for separating 740 adjacent test strips 705 from one another as well as means for separating 740 test strips 705 from the surround area 742. The means for separating 740 are preferably perforations in the edges of adjacent test strips that are only joined at connecting points 740. Each test strip extends between a first end 710 and a second end 720. Each test strip 705 has a reagent test portion (not illustrated in FIG. 7) and also includes a recloseable cover 730. The card 700 preferably has a height and a width corresponding to those of a credit card. Each test strip 705 has dimensions corresponding to existing test strips (ACCU-CHECK® Aviva strips) manufactured by Roche Diagnostics Operations Inc., the assignee of the present application, having a thickness 707 of approximately 9 mm and a standard length 717 of about 38 mm. The card 700 is laid out as six rows with two strips in each row, the count of the card illustrated in FIG. 7 being twelve test strips. The reagent portions of each strip 705 are substantially adjacent to one another, but other configurations are contemplated as within the scope of the invention. For example, the strips 705 can be loaded in an opposite layout in which the reagent end is at the outside edge.

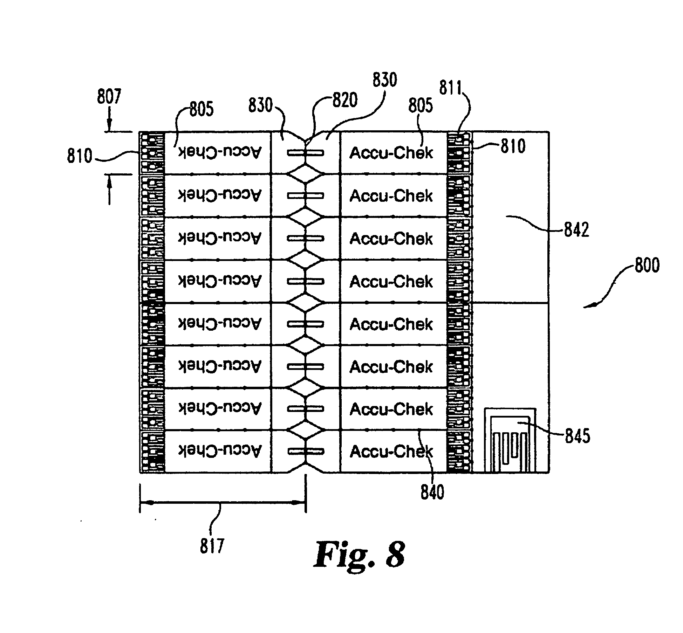

With reference to FIG. 8 there is illustrated another embodiment of a test strip card 800 having a plurality of electrochemical biosensor test strips 805 in a two dimensional array. In this embodiment the card 800 includes a surround area 842 at only one end of the card 800. The card 800 includes means for separating 840 adjacent test strips 805 from one another as well as means for separating 840 test strips 805 from the surround area 842. The means for separating 840 are preferably perforations in the edges of adjacent test strips that are only joined at connecting points 840. Each test strip extends between a first end 810 and a second end 820. Each test strip 805 has a reagent test portion (not illustrated in FIG. 8) and also includes a recloseable cover 830. The card 800 preferably has a height and a width corresponding to those of a credit card. Each test strip 805 has dimensions corresponding to existing test strips (ACCU-CHECK® Performa strips) manufactured by Roche Diagnostics Operations Inc., the assignee of the present application, having a thickness 807 of approximately 7 mm and a standard length 817 of about 38 mm. The card 800 is laid out as eight rows with two strips in each row, the count of the card illustrated in FIG. 8 being sixteen test strips. The reagent portions of each strip 805 are substantially adjacent to one another, but other configurations are contemplated as within the scope of the invention. The surround area 842 might include any of a number of refinements previously discussed that include, but not limited to, the illustrated data storage device 845.

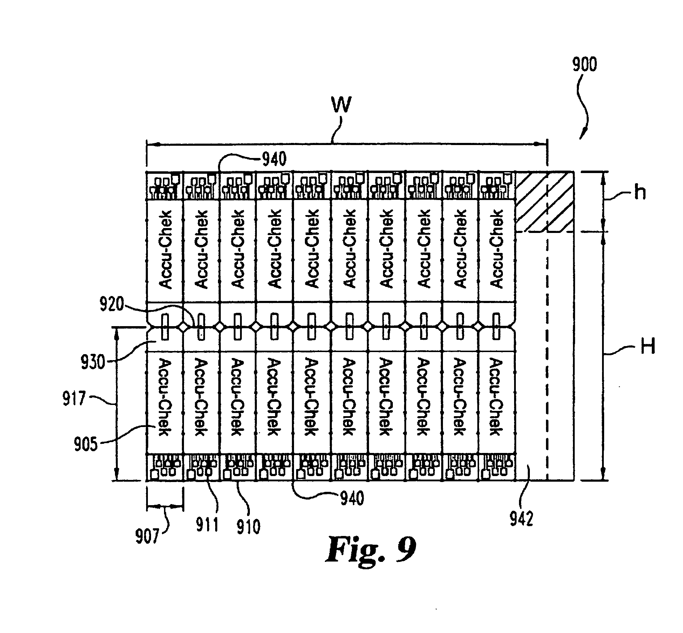

With reference to FIG. 9 there is illustrated another embodiment of a test strip card 900 having a plurality of electrochemical biosensor test strips 905 in a two dimensional array. In this embodiment the card 900 includes a surround area 942 at only one end of the card 900. The card 900 includes means for separating 940 adjacent test strips 905 from one another as well as means for separating 940 test strips 905 from the surround area 942. The means for separating 940 are preferably perforations in the edges of adjacent test strips that are only joined at connecting points 940. Each test strip extends between a first end 910 and a second end 920. Each test strip 905 has a reagent test portion (not illustrated in FIG. 9) and also preferably includes a recloseable cover 930.

The card 900 preferably has a height and a width approximately that of a credit card. Each test strip 905 has dimensions corresponding to existing test strips (ACCU-CHECK® Performa strips) manufactured by Roche Diagnostics Operations Inc., the assignee of the present application, having a thickness 907 of approximately 7 mm and a standard length 917 of about 38 mm. As illustrated in FIG. 9, a credit card has a width W and a height H, such that the height of card 900 is that of two strips 905 laid end to end is a distance h of about 15 mm greater than the standard credit card height H. Alternatively, to more closely approximate the size of a credit card, the length of each strip 905 might be trimmed by about 7 mm from the current 38 mm length to a length of about 30 or 31 mm. The card 900 is laid out as ten rows with two strips in each row, the count of the card illustrated in FIG. 9 being twenty test strips. The reagent portions of each strip 905 are substantially adjacent to one another, but other configurations are contemplated as within the scope of the invention. The surround area 942 might include any of a number of refinements previously discussed.

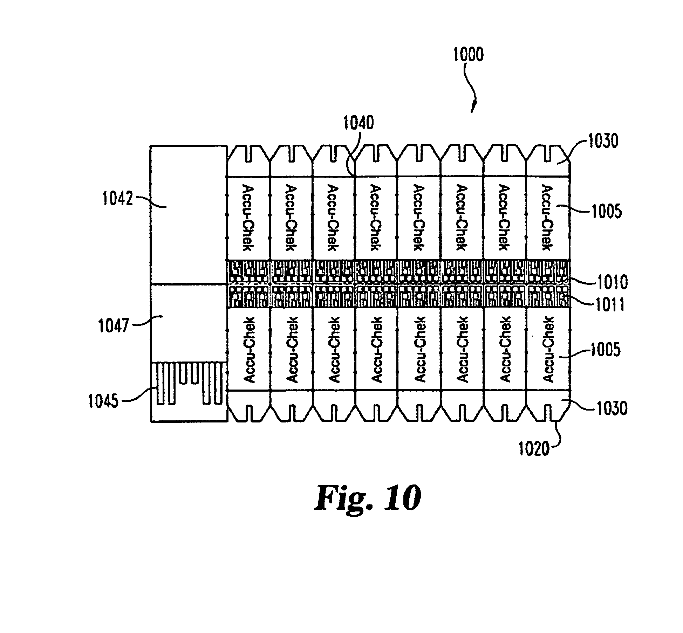

The card 900 has a two dimensional array of 7 mm wide strips 905 in a vertical layout. A two dimensional array of 9 mm strips having a shortened length in a similar vertical layout yields eight rows of two strips resulting in a sixteen count card. Such a card is illustrated in FIG. 10. It should be understood that variations of both FIGs. 9 and 10 are contemplated as within the scope of the invention. For example, the length of the strips could be further shortened permitting the surround area to extend along the width of the card 900 or 1000 while still approximating the size of a standard credit card.

With reference to FIG. 10 there is illustrated another embodiment of a test strip card 1000 having a plurality of electrochemical biosensor test strips 1005 in a two dimensional array. In this embodiment the card 1000 includes a surround area 1042 at only one end of the card 1000. The card 1000 includes means for separating 1040 adjacent test strips 1005 from one another as well as means for separating 1040 test strips 1005 from the surround area 1042. The means for separating 1040 are preferably perforations in the edges of adjacent test strips that are only joined at connecting points 1040. Each test strip extends between a first end 1010 and a second end 1020. Each test strip 1005 has a reagent test portion (not illustrated in FIG. 10) and also preferably includes a recloseable cover 1030.

The card 1000 preferably has a height and a width approximately that of a credit card. Each test strip 1005 has dimensions corresponding to a shortened length version of existing test strips (ACCU-CHECK® Performa strips) manufactured by Roche Diagnostics Operations Inc., the assignee of the present application, having a thickness of approximately 7 mm. The card 1000 is laid out as eight rows with two strips in each row, the count of the card illustrated in FIG. 10 being sixteen test strips. The reagent portions of each strip 1005 are on the outside edge of the card, but other configurations are contemplated as within the scope of the invention. The surround area 1042 might include any of a number of refinements previously discussed. For example, the surround area 1042 is illustrated as including a data storage device 1045 as well as space 1047 that might include a logo or other information.

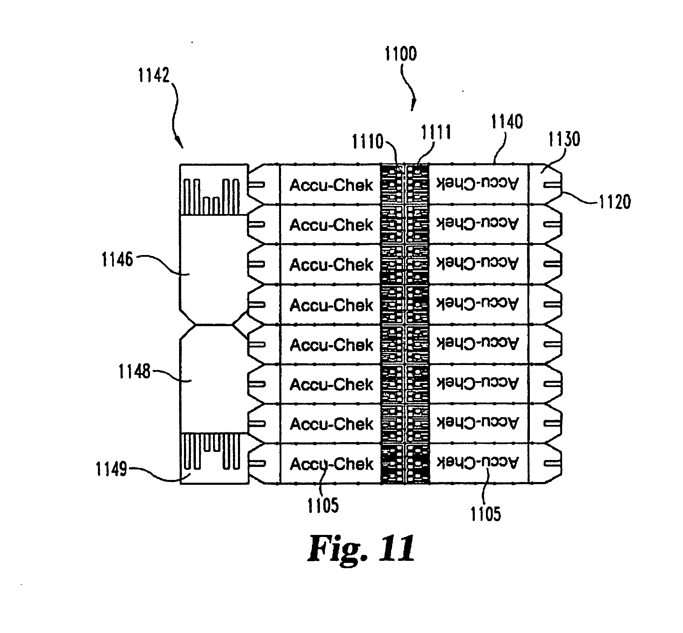

With references to FIG. 11 there is illustrated a 16 count option test strip card that includes both a data storage device and a BIT activated feature set key. Test strip card 1100 has a plurality of electrochemical biosensor test strips 1105 in a two dimensional array. In this embodiment the card 1100 includes a surround area 1142 at only one end of the card 1100. The card 1100 includes means for separating 1140 adjacent test strips 1105 from one another as well as means for separating 1140 test strips 1105 from the surround area 1142. The means for separating 1140 are preferably perforations in the edges of adjacent test strips that are only joined at connecting points 1140. Each test strip extends between a first end 1110 and a second end 1120. Each test strip 1105 has a reagent test portion (not illustrated in FIG. 11) and also preferably includes a recloseable cover 1130.

The card 1100 preferably has a height and a width approximately that of a credit card. Each test strip 1105 has dimensions corresponding to a shortened or standard length version of existing test strips (ACCU-CHECK® Performa strips) manufactured by Roche Diagnostics Operations Inc., the assignee of the present application, having a thickness of approximately 7 mm. The card 1100 is laid out as eight rows with two strips in each row, the count of the card illustrated in FIG. 11 being sixteen test strips. The reagent portions of each strip 1105 are on the outside edge of the card, but other configurations are contemplated as within the scope of the invention. The surround area 1142 might include any of a number of refinements previously discussed. For example, the surround area 1142 is illustrated as including a data storage device 1145 as well as a BIT activated feature key 1148 and further includes the option of having a slip cover for protection at area 1149.

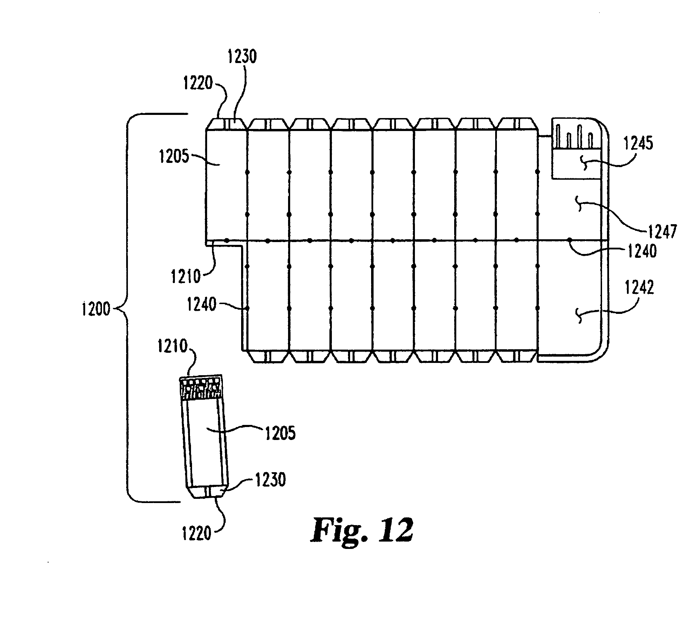

With reference to FIG. 12 there is illustrated another embodiment of a test strip card 1200 having a plurality of electrochemical biosensor test strips 1205 in a two dimensional array, with one test strip 1205 illustrated as detached from the card 1200. In this embodiment the card 1200 includes a surround area 1242 at only one end of the card 1200. The card 1200 includes means for separating 1240 adjacent test strips 1205 from one another as well as means for separating 1240 test strips 1205 from the surround area 1242. The means for separating 1240 are preferably perforations in the edges of adjacent test strips that are only joined at connecting points 1240. Each test strip extends between a first end 1210 and a second end 1220. Each test strip 1205 has a reagent test portion (not illustrated in FIG. 12) and also preferably includes a recloseable cover 1230.

The card 1200 preferably has a height and a width approximately that of a credit card. Each test strip 1205 preferably has dimensions corresponding to a shortened length version of existing test strips (ACCU-CHECK® Performa strips) manufactured by Roche Diagnostics Operations Inc., the assignee of the present application, having a thickness of approximately 7 mm. The card 1200 is laid out as eight rows with two strips in each row, the count of the card illustrated in FIG. 12 being sixteen test strips. The reagent portions of each strip 1205 are on the outside edge of the card, but other configurations are contemplated as within the scope of the invention. The surround area 1242 might include any of a number of refinements previously discussed. For example, the surround area 1242 is illustrated as including a data storage device 1245 as well as space 1247 that might include a logo or other information.

Instead of being patterned after the size of a credit card, the test strip card could be patterned after the size of the analyte meter. In various embodiments the test strip card might be sized and shaped to be carried, concealed and/or retained on the back of the analyte meter. It will be understood that the meter might be configured to retain multiple test strip cards, or even a shrink wrapped pack of test strip cards. In one embodiment the analyte meter is a blood glucose meter, however meters for other analytes are contemplated as within the scope of the invention.

With reference to FIG. 13 there is illustrated an embodiment of a test strip card 1300 in which the sheet, or combination of sheet and retaining platform might possess a size and shape different from a credit card. Test strip card 1300 is sized to be retained on the back face of the analyte meter 1310. As illustrated the meter 1310 includes at least one flange or tab 1350 to retain the test strip card 1300 to the meter 1310. In one embodiment the meter 1310 includes two tabs 1350. The meter 1310 preferably includes a retaining "ball" or other feature 1360 to keep pressure on the test strip card(s) 1300 to prevent the card(s) from falling out.

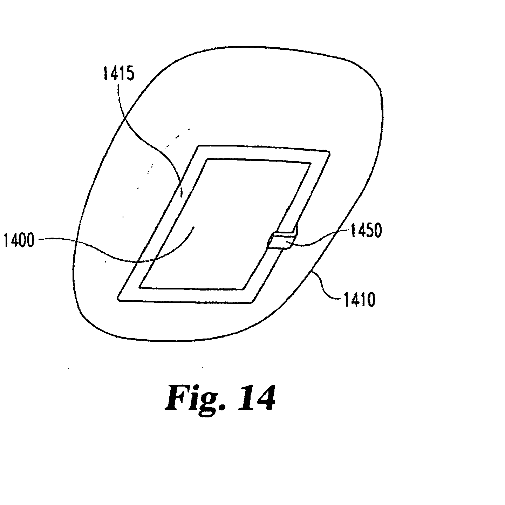

With reference to FIG. 14 there is illustrated another embodiment of a test strip card 1400 in which the sheet, or combination of sheet and retaining platform might possess a size and shape different from a credit card. Test strip card is sized to be retained on the back face of the analyte meter 1410. Meter 1410 has an internal recess or slot 1415 therein for receiving test strip card(s) 1400. As illustrated the meter 1410 include at least one flange or tab 1450 to retain the test strip card(s) 1400 to the meter 1410. Again, the meter 1410 preferably includes a retaining "ball" or other feature (not illustrated) to keep pressure on the test strip card(s) 1400 to prevent the card(s) from falling out.

While the invention has been illustrated and described in detail in the drawings and foregoing description, the same is to be considered as illustrative and not restrictive in character, it being understood that only the preferred embodiments have been shown and described. It should be understood that while the use of words such as preferable, preferably, preferred or more preferred utilized in the description above indicate that the feature so described may be more desirable, it nonetheless may not be necessary and embodiments lacking the same may be contemplated as within the scope of the invention, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. An analyte test strip card comprising:
A sheet having a plurality of test strips, each test strip being detachably connected to adjacent test strips via means for separation,
**characterized by**
each test strip also having at least a portion of an edge detachably connected to a retaining platform via means for separation from the retaining platform said retaining platform extending around at least a portion of a platform
perimeter of the sheet, wherein the retaining platform provides a surround area that defines the overall size.

2. The card of claim 1, wherein the platform is U-shaped.

3. The card of claim 1, wherein the platform includes a ROM key.

4. The card of claim 3, wherein the card is one of a plurality of cards stacked atop one another, and wherein the stack of cards is enclosed within an air tight shrink seal.

5. The card of claim 1, wherein each of the plurality of strips has a corresponding recloseable cover overlapping a reagent portion of the strip.

6. The card of claim 1, wherein the card is substantially the size of a credit card.

7. The card of claim 6, wherein the plurality of strips are a two dimensional array of electrochemical biosensor test strips for measuring blood glucose.

8. The card of claim 1, wherein the plurality of strips are a two dimensional array.

9. The card of claim 1, in combination with a blood glucose meter having a back face, wherein the card is a blood glucose test strip card having a size permitting retention within the back face of the meter.

## Patentansprüche

1. Analyt-Teststreifenkarten, umfassend:
eine Platte mit einer Vielzahl von Teststreifen, wobei jeder Teststreifen über ein Trennmittel abnehmbar mit benachbarten Teststreifen verbunden ist,
**dadurch gekennzeichnet, dass**
zumindest ein Teil eines Randes jedes Teststreifens über ein Mittel zur Trennung von der Halteplattform abnehmbar mit einer Halteplattform verbunden ist, wobei sich die Halteplattform zumindest um einen Teil eines Umfangs der Platte erstreckt, worin die Halteplattform einen Umgebungsbereich bereitstellt, der die Gesamtgröße definiert.

2. Karte nach Anspruch 1, worin die Plattform U-förmig ist.

3. Karte nach Anspruch 1, worin die Plattform einen ROM-Schlüssel aufweist.

4. Karte nach Anspruch 3, worin die Karte eine von einer Vielzahl von aufeinander gestapelten Karten ist und worin der Kartenstapel in einer luftundurchlässigen Schrumpfdichtung eingeschlossen ist.

5. Karte nach Anspruch 1, worin die Vielzahl von Streifen eine entsprechende wiederverschließbare Abdeckung aufweist, die einen Reagenzabschnitt des Streifens überlappt.

6. Karte nach Anspruch 1, worin die Karte im Wesentlichen die Größe einer Kreditkarte aufweist.

7. Karte nach Anspruch 6, worin die Vielzahl von Streifen eine zweidimensionale Anordnung von elektrochemischen Biosensor-Teststreifen zur Messung von Blutzucker ist.

8. Karte nach Anspruch 1, worin die Vielzahl von Streifen eine zweidimensionale Anordnung ist.

9. Karte nach Anspruch 1, in Kombination mit einem Blutzuckermessgerät mit einer Rückseite, worin die Karte eine Blutzucker-Teststreifenkarte ist, deren Größe es erlaubt, dass sie in der Rückseite des Messgeräts gehalten wird.

## Revendications

1. Carte à bandelettes réactives pour un analyte comprenant :
une plaque ayant une pluralité de bandelettes réactives, chaque bandelette réactive étant reliée de manière détachable à des bandelettes réactives adjacentes par des moyens de séparation,
**caractérisée par** chaque bande réactive ayant également au moins une partie d'un bord reliée de manière détachable à une plateforme de retenue par des moyens de séparation de la plateforme de séparation, ladite plateforme de séparation s'étendant autour d'au moins une partie d'un périmètre de la plaque, dans laquelle la plateforme de retenue fournit une zone d'entourage qui définit la taille globale.

2. Carte selon la revendication 1, dans laquelle la plateforme est en forme de U.

3. Carte selon la revendication 1, dans laquelle la plateforme comprend une carte ROM.

4. Carte selon la revendication 3, dans laquelle la carte est l'une de la pluralité de cartes empilées les unes sur les autres, et dans laquelle la pile de cartes est enfermée dans un film plastique rétrécissable scellé étanche à l'air.

5. Carte selon la revendication 1, dans laquelle chacune de la pluralité de bandelettes a un couvercle correspondant pouvant être refermé chevauchant une partie réactive de la bandelette.

6. Carte selon la revendication 1, dans laquelle la carte a essentiellement la taille d'une carte de crédit.

7. Carte selon la revendication 6, dans laquelle la pluralité de bandelettes forme un réseau bidimensionnel de bandelettes réactives de biocapteurs électrochimiques pour mesurer la glycémie.

8. Carte selon la revendication 1, dans laquelle la pluralité de bandelettes forme un réseau bidimensionnel.

9. Carte selon la revendication 1, en combinaison avec un lecteur de glycémie ayant une face arrière, dans laquelle la carte est une carte à bandelettes de test de glycémie ayant une taille permettant la retenue à l'intérieur de la face arrière du lecteur.
